# EUROPEAN PATENT SPECIFICATION

(11) **EP 2 197 892 B1**
(45) Date of publication and mention of the grant of the patent: **16.07.2014**
(21) Application number: 08805204.8
(22) Date of filing: 10.10.2008
(51) Int. Cl.: C07H 19/00, C07H 19/06, C07H 19/12, C07H 19/16

(54) **METHOD OF PRODUCING NUCLEOSIDES**
VERFAHREN ZUR HERSTELLUNG VON NUKLEOSIDE
PROCÉDÉ DE PRÉPARATION DE NUCLÉOSIDES

(30) Priority: 10.10.2007 EP 07019825
(43) Date of publication of application: 23.06.2010
(73) Proprietor: Cilag AG, 8205 Schaffhausen (CH)
(72) Inventor: JUNGMANN, Oliver, 78166 Donaueschingen (DE); KRAUT, Norbert, 78250 Tengen (DE)
(74) Representative: Verberckmoes, Filip Gerard
(86) International application number: PCT/EP2008/063582
(87) International publication number: WO 2009/047314

(56) References cited:
- US-A- 4 082 911
- US-A- 4 209 613
- US-A- 5 811 540
- LU K-C ET AL: "Simple and efficient per-O-acetylation of carbohydrates by lithium perchlorate catalyst", TETRAHEDRON, ELSEVIER SCIENCE PUBLISHERS, AMSTERDAM, NL, vol. 60, no. 40, 27 September 2004 (2004-09-27), pages 8967-8973, XP004565047, ISSN: 0040-4020, DOI: 10.1016/J.TET.2004.06.138

## Description

The present invention refers to a method of producing nucleosides, the compound 2'-deoxy-5-azacytidine (Decitabine) being excluded, by reacting a protected, preferably silylated, nucleoside base with a glycoside donor, preferably a 1-halogen derivative, or 1-0-acyl, 1-0-alkyl, or an imidate preferably a trichloromethyl imidate, or a thio-alkyl derivative of a blocked monosaccharide or oligosaccharide, preferably ribose and 2-desoxyribose derivatives, in the presence of a selected catalyst.

### State of the Art

Nucleosides are known pharmaceutically active compounds and have been described in numerous publications. From US 3,817,980 it is known to synthesize nucleosides by silylating the corresponding nucleoside base and reacting the silylated base with a glycosyl donor preferably a 1-halogen derivative of a blocked monosaccharide or oligosaccharide in the presence of a selected catalyst. The catalysts used are e.g. selected from SnCl₄, TiCl₄, ZnCl₂, BF₂-etherate, AlCl₃ or SbCl₅. The major disadvantage is that these catalysts are prone to hydrolysis giving irritant hydrolysis products like HCl and/or are forming insoluble oxides (TiO₂, SnO₂), which are difficult to remove from the reaction product. These catalysts and difficult to handle, especially on large scale production.

US-A-4 082 911 refers to the analogous process of reacting a silylated nucleoside base with a protected derivative of a sugar and proposes to use as catalyst a trialkylsilyl ester of a strong organic acid, such as trimethylsilyl-trifluoromethanesulfonate. US-A-4 209 613 proposes an improvement for the method disclosed in US-A-4 082 911 by using a single-step process wherein the trialkylsilyl ester of the strong organic acid, such as trimethylsilyl-trifluoromethanesulfonate, is formed in *situ* from the free acid by reaction of the free acid with the silylating agent, e.g. trialkylchlorosilane, which is present in the appropriate molar amount. Silylating agents such as trialkylchlorosilane, are very reactive and quickly react to form the trialkylsilyl ester of the free acid present in the reaction mixture.

### Description of the invention

It has now been found that a glycoside donor preferably a 1-halogen derivative, or 1-0-acyl, 1-0-alkyl, or an imidate, preferably a trichloromethyl imidate [-NH-(O)C-CCl₃], or a thio-alkyl derivative of a blocked monosaccharide or oligosaccharide, preferably ribose and 2-desoxyribose derivatives, can be reacted with a silylated or alkylated nucleoside base in the presence of a selected catalyst, said catalyst being a salt of an aliphatic sulphonic acid. There is no need to use an ester compound as a catalyst. This very much simplifies the production of nucleosides as described in the present invention. This type of catalyst is stable under aqueous conditions, easy to handle, does not produce irritant hydrolysis products, and can be easily removed.

Furthermore, using the catalyst of the present invention, the selectivity of the reaction for obtaining the desired anomer, i.e. the ratios of the alpha/beta anomers are excellent. Further, according to the present invention a reaction yield that is higher than 95%, and regularly is within the range of 97-99%, calculated to the total amount of anomers present in the final crude reaction mixture, can be obtained.

The present invention is defined in the claims. The present invention refers to a method of producing a free nucleoside compound, with the proviso that the compound 2'-deoxy-5-azacytidine (Decitabine) is excluded, by reacting a glycoside donor, preferably a 1-halogen, or 1-0-acyl, or a 1-0-alkyl, or an imidate, preferably a trichloromethyl imidate derivative, or a thio-alkyl derivative, preferably a thiomethyl derivative, of a blocked monosaccharide or oligosaccharide, preferably a ribose and/or a 2-desoxyribose derivative, said monosaccharide or oligosaccharide being blocked by removable protecting groups, with a protected nucleoside base, in a suitable anhydrous solvent and in the presence of a catalyst, whereby a blocked nucleoside compound is obtained, and removing the protecting groups from said blocked nucleoside compound in order to obtain the free nucleoside compound, characterized in that said catalyst is a salt of an aliphatic sulphonic acid.

The present invention refers also to the production of the blocked nucleoside compound, with the exception of the blocked compound of 2'-deoxy-5-azacytidine (Decitabine), as obtained from the reaction of the glycoside donor preferably a 1-halogen derivative, or 1-0-acyl, or 1-0-alkyl, or an imidate, preferably a trichloromethyl imidate derivative, or a thio-alkyl derivative, preferably a thiomethyl derivative, of a blocked monosaccharide or oligosaccharide, preferably a ribose and/or a 2-desoxyribose derivative, with the protected nucleoside base, characterized in that in said reaction the catalyst is a salt of an aliphatic sulphonic acid.

The catalyst used in said reaction as a salt of an aliphatic sulphonic acid preferably is preferably a salt of methylsulphonic acid (mesylate) or of ethylsulphonic acid, or is a salt of a fluorinated aliphatic sulfonic acid, such as a salt of trifluoromethane-sulfonic acid, of pentafluoroethyl-sulfonic acid, or of heptafluoropropyl-sulfonic acid.

Preferred of these salts are the salts of methylsulphonic acid (mesylate), and the salts of trifluoromethanesulfonic acid.

Preferred aliphatic sulphonic acid salts, and fluorinated aliphatic sulfonic acid salts are the alkali salts and earth alkali salts, preferably the salts of lithium, sodium, potassium, or magnesium. Preferred are the lithium salts, preferably lithium methylsulphonic acid (lithium mesylate), and lithium-trifluoromethanesulfonate (LiOTf, lithiumtriflate). Also other salts, for example the salts of scandium, such as Sc(OTf)₃ or of copper such as Cu(OTf)₂ or of magnesium such as Mg(OTf)₂ can be used. However, the lithium salt and especially LiOTf is preferred.

Preferred solvents to carry out the reaction according to the present invention are organic solvents such as benzene, toluene, xylol, or chlorinated solvents, for example dichloromethane, dichloroethane, chloroform, chlorobenzene, or acetonitril and/or propylene carbonate and/or related solvents. Preferred are toluene and chlorinated solvents. Preferred is the use of lithium-trifluoromethanesulfonate (LiOTf) in a chlorinated solvent, preferably in dichloromethane, dichloroethane, chloroform, chlorobenzene and/or in an aromatic solvent like toluene or xylene. Each solvent or mixture of solvents may yield a different selectivity with respect to the beta-isomer (β-isomer). It is no problem for the expert in the art to optimize the catalyst and/or solvent or the mixture of solvents in order to obtain the desired selectivity in favor of the beta-isomer.

The synthesis of glycoside donors as defined herein is known per se. The glycoside donor preferably is a 1-halogen derivative, or 1-O-acyl, or a 1-0-alkyl, or an imidate preferably a trichloromethyl imidate derivative, or a thio-alkyl derivative of a blocked monosaccharide or oligosaccharide, preferably of a blocked ribose and/or 2-desoxyribose, wherein the hydroxyl groups are being blocked by protecting groups, i.e. removable substituents. Such compounds and numerous substituents are known and can be used within the scope of the present invention.

Said protecting groups preferably are selected from (C₁-C₈)alkylcarbonyl, or optionally substituted phenylcarbonyl, or optionally substituted benzylcarbonyl. Preferably, said protecting groups are selected from (C₁-C₄)alkylcarbonyl, or optionally substituted phenylcarbonyl, like phenylcarbonyl, tolylcarbonyl, xylylcarbonyl or benzylcarbonyl; and is preferably acetyl or p-chloro-phenylcarbonyl.

The substituents 1-0-acyl, 1-0-alkyl, 1-halogen, 1-imidate or 1-thio-alkyl attached to the blocked monosaccharide or oligosaccharide are preferably substituents of the formulae -O-(C₁-C₈) acyl, -O-(C₁-C₈)alkyl or halogen or trichloromethyl imidate, or thiomethyl; preferably are -O-(C₁-C₄)acyl, -O-(C₁-C₄)alkyl or chlorine, bromine, fluorine; preferably -O-C(O)-CH₃ or chlorine, bromine, fluorine, preferably chlorine or fluorine, preferably chlorine.

The blocked monosaccharide or oligosaccharide are preferably derived from ribose, deoxyribose, arabinose, and glucose, preferably from ribose and 2-desoxyribose. Preferably all free hydroxyl groups are blocked with known protecting groups, preferably with the blocking groups as mentioned herein above, selected from (C₁-C₈)alkylcarbonyl, or optionally substituted phenylcarbonyl, or optionally substituted benzylcarbonyl. These blocking groups are known to the expert in the art.

The protected nucleoside base is protected by a removable protecting group known per se, and is preferably protected by a trimethylsilyl (TMS)-group. The preparation of protected nucleoside base compounds is known. The compound is preferably prepared by reaction of the free nucleoside base with trimethylchlorosilane or with hexamethyldisilazane. This is known to the expert in the art. Numerous nucleoside organic bases are known. Generally all these nucleoside organic bases can be reacted with the corresponding chemical compounds, e.g. trimethylchlorosilane or with hexamethyldisilazane, to yield the protected nucleoside base which can be used according to the process defined in the present invention.

Preferred nucleoside bases are halogen-derivatives, preferably chloro- or fluoro-substituted derivatives, preferably fluorine substituted. Preferably the nucleoside bases are or are derived from the group of uracil, cytosine, preferably 5-azacytosine, 6-azauracil, 2-thio-6-azauracil, thymine, and guanine. In the reaction of the nucleoside base with the glycoside donor, the sugar residue is preferably linked to the nitrogen atom to form a beta-glycoside.

When reacting the protected nucleoside base with the above mentioned derivative of a blocked monosaccharide or oligosaccharide together, the reaction temperature generally is within the range of 0°C to about 90°C, preferably at about room temperature, whereby the components are reacted in about equimolar amounts and preferably with a slight excess of the protected nucleoside base. The catalyst is used preferably in a concentration of about 10 to 100 mol-%, calculated to the total molar presence of the two reacting components. For the expert in the art it is no problem to optimize the molar ratios of the components.

For removing the substituents from the blocked nucleoside compound in order to obtain the free nucleoside compound, containing free hydroxyl groups, known methods are used. The substituents may be preferably removed, for example, by treatment in an alcoholic solution of ammonia or alcoholates, or with aqueous or alcoholic alkali; but other known methods may be applied. The following example illustrates the invention.

### Example 1

(A) A mixture of cytosine (2.5 g, 22.5 mmol), ammonium sulfate (0.30 g, 2.27 mmol), and hexamethyldisilazane (20.0 g, 123.9 mmol) was heated to reflux for 17 h. A clear solution was obtained. Some gas evolved (ammonia). The reaction mixture was cooled to 52°C and concentrated in the vacuum whereby a colourless solid precipitated. 25 ml of dichloromethane, lithium trifluoromethane sulfonate (3.51 g, 22.5 mmol) and 1-chloro-3,5di-o-*p*-chlorobenzoyl-2-deoxy-α-D-ribofuranose (9.67 g, 22.5 mmol) were added. The slightly beige mixture was stirred for 17 hours at ambient temperature (20-25°C), products (reaction yield combined anomers: 99.1%; selectivity alpha/beta 38.5/61.5)

(B) Then the solvent was removed at 38°C. A brownish solid was obtained. The solid was dissolved in 7.5 g ethyl acetate. The solution was added drop wise to a mixture of 27.5 g of aqueous sodium hydrogen carbonate (2.5 weight% solution in water), 21.8 g of ethyl acetate, 4.5 g of cyclohexane and 8.8 g of acetonitrile at 20°C. The obtained reaction mixture was stirred at ambient temperature for 1 h, and then for 1.5 hours at 0°C. The precipitate of the blocked (protected) nucleoside was filtered off, washed with 10g of water, and finally with 20 g of a mixture of acetonitrile and ethyl acetate (1:1).
Yield: 9.90 g; 87.2% combined anomers; ratio alpha/beta 41/59; purity 98.83%.

### Example 2

(A) A mixture of 5-fluoro-cytosine (1.0 g, 7.75 mmol), ammonium sulfate (0.12 g, 0.91 mmol), and hexamethyldisilazane (8.0 g, 49.6 mmol) was heated to reflux for 17 hours. A clear solution was obtained. Some gas evolved (ammonia). The reaction mixture was cooled to 50°C, and concentrated in the vacuum to about half of the original volume. A turbid suspension was obtained. 10 ml of dichloromethane, lithium trifluoromethane sulfonate (1.21 g, 7.75 mmol) and 1-chloro-3,5-di-o-*p*-chlorobenzoyl-2-deoxy-α-D-ribofuranose (3.33 g, 7.75 mmol) were added. The beige mixture was stirred for 17 hours at ambient temperature (20-25°C) (reaction yield combined anomers: 99.0%; selectivity alpha/beta 30/70]).
(B) Then the solvent was removed at 38°C. A brownish sticky solid was obtained. The solid was dissolved in 10 g ethyl acetate. The solution was added to 10 g of aqueous hydrogen carbonate (2.5 weight% solution in water). The reaction mixture was stirred at ambient temperature for 2.5 hours, and then filtered off, washed with 3.0 g of water, and finally with 3.6 g ethyl acetate
Yield: 1.80 g; 44.5%; alpha/beta 1.2/98.8; purity: 98.5% beta anomer.

### Example 3

(A) A mixture of 5-azacytosine (0.25 g, 2.23 mmol), ammonium sulfate (0.03 g, 0.23 mmol), and hexamethyldisilazane (2.0 g, 12.4 mmol) was heated to reflux for 17 hours. A clear solution was obtained. Some gas evolved (ammonia). The reaction mixture was cooled to 50°C, and concentrated in the vacuum to about half of the original volume. A turbid suspension was obtained. 3.3 g of dichloromethane, lithium trifluoromethane sulfonate (0.35 g, 2.24 mmol) and 2,3,5-tri-O-benzoyl-alpha-D-arabinofuranosylbromide (1.17 g, 2.23 mmol) were added. The beige mixture was stirred for 17 hours at ambient temperature (20-25°C) (reaction yield: 95.2%; selectivity: 0.1/99.9).
(B) Then the solvent was removed at 38°C. A brownish sticky solid was obtained. The solid was dissolved in 7.0 g ethyl acetate and the reaction mixture was washed with 7.0 g of aqueous hydrogen carbonate (2.5 weight% solution in water). The organic phase was filtered, dried over Na₂SO₄ and evaporated under vacuum. The residue was dissolved in 3.0 g ethyl acetate, 4.0 g cyclohexane was added and the mixture was stirred for 2 hours at ambient temperature. The crystallized product was filtered off and washed with a small amount of a 1:1 mixture of ethyl acetate and cyclohexane.
Yield: 0.40 g; 32.2%; purity: 99.2%

### Example 4

(A) A mixture of 5-Azacytosine (0.25 g, 2.23 mmol), ammonium sulfate (0.03 g, 0.23 mmol), and hexamethyldisilazane (2.0 g, 12.4 mmol) was heated to reflux for 17 hours. A clear solution was obtained. Some gas evolved (ammonia). The reaction mixture was cooled to 50°C, and concentrated in the vacuum to about half of the original volume. A turbid suspension was obtained. 3.3 g of dichloromethane, lithium trifluoromethane sulfonate (0.35 g, 2.24 mmol) and 2,3,5-Tri-O-benzoyl-alpha-D-arabinofuranosylbromide (1.17 g, 2.23 mmol) were added. The beige mixture was stirred for 17 hours at ambient temperature (20-25°C) (Reaction Yield: 94.2%).
(B) The solvent was removed at 38°C. To the oily residue 4.0 g of ethyl acetate were added and then the turbid solution was filtered. The filtrate was quenched with 4.0 g aqueous sodium hydrogen carbonate solution (2.5%-weight). Afterwards 4.0 g of cyclohexane were added and the mixture was cooled to 0-5°C for 4 hours. The solid was filtered off (5-Azacytosine) and washed with a mixture of ethyl acetate and cyclohexane (1:1). The filtrate was put in a separatory funnel and the product containing organic phase was separated and dried over sodium sulfate. Afterwards the solvent was removed under vacuum and diethyl ether was added to the oily residue, whereas a solid was formed. After stirring for 24 hours the precipitation was filtered off and washed with diethyl ether.
Yield: 0.70 g; 56.3%; Purity: 91.5%

### Example 5

Examples 1 to 4 are repeated replacing lithium trifluoromethane sulfonate each time by the same amount (in equivalents) of one of the compounds lithium mesylate, lithium tetrafluroborate, sodium trifluoromethane sulfonate, potassium trifluoromethane sulfonate and zinc trifluoromethane sulfonate, whereby analogous results are obtained as reported in Examples 1-4.

### Example 6

Examples 1 to 4 are repeated replacing dichloromethane as solvent each time by the same volume of one of the following solvents: toluene, xylene, 1,2-dichloroethane, chlorobenzene, 1,2-dichlorobenzene, acetonitrile and propylenecarbonate, whereby analogous results are obtained as reported in Examples 1-4.

## Claims

1. Method of producing a free nucleoside compound, with the proviso that the compound 2'-deoxy-5-azacytidine (Decitabine) is excluded, by reacting a glycoside donor, preferably a 1-halogen derivative, or 1-O-acyl, 1-0-alkyl, or an imidate, preferably a trichloromethyl imidate, or a thio-alkyl derivative preferably a thiomethyl derivative of a blocked monosaccharide or oligosaccharide, preferably ribose and 2-desoxyribose derivatives, said monosaccharide or oligosaccharide being blocked by removable protecting groups, with a protected nucleoside base, in a suitable anhydrous solvent and in the presence of a catalyst, whereby a blocked nucleoside compound is obtained, and removing the protecting groups from said blocked nucleoside compound in order to obtain the free nucleoside compound, **characterized in that** said catalyst is a salt of an aliphatic sulphonic acid or a salt of a fluorinated aliphatic sulphonic acid.

2. Method of producing a blocked nucleoside compound, with the proviso that the blocked compound of 2'-deoxy-5-azacytidine (Decitabine) is excluded, by reacting a glycoside donor, preferably a 1-halogen derivative, or 1-0-acyl, 1-0-alkyl, or an imidate preferably a trichloromethyl imidate, or a thio-alkyl derivative of a blocked monosaccharide or oligosaccharide, preferably ribose and 2-desoxyribose derivatives, said monosaccharide or oligosaccharide being blocked by removable protecting groups, with a protected nucleoside base, in a suitable anhydrous solvent and in the presence of a catalyst, whereby a blocked nucleoside compound is obtained, **characterized in that** said catalyst is a salt of an aliphatic sulphonic acid or a salt of a fluorinated aliphatic sulphonic acid.

3. Method according to claim 1 or 2, **characterized in that** the catalyst used in said reaction is a salt of methylsulphonic acid or of ethylsulphonic acid, or a salt of trifluoromethane-sulfonic acid, pentafluoroethyl-sulfonic acid, or heptafluoropropyl-sulfonic acid.

4. Method according to claim 3, **characterized in that** the catalyst is a salt of methylsulphonic acid and/or a salt of trifluoromethanesulfonic acid.

5. Method according to any one of the claims 1-4, **characterized in that** the catalyst is an alkali salt or an earth alkali salt, preferably a salt of lithium, sodium, potassium, or magnesium, preferably a lithium salt.

6. Method according to any one of the claims 1-5, **characterized in that** the catalyst is lithium methylsulphonic acid and/or lithium-trifluoromethanesulfonate.

7. Method according to claim 1-4, **characterized in that** the catalyst is chosen from the salts comprising salts of scandium, preferably Sc(OTf)₃, or of copper preferably Cu(OTf)₂ or of magnesium, preferably Mg(OTf)₂.

8. Method according to any one of the claims 1-7, **characterized in that** the solvent to carry out the reaction is chosen from the group comprising organic solvents, preferably benzene, toluene, xylene, or chlorinated solvents, preferably dichloromethane, dichloroethane, chloroform, chlorobenzene, or toluene, xylol, or acetonitril, propylene carbonate and related solvents,

9. Method according to claim 8, **characterized in that** the solvent to carry out the reaction is chosen from organic solvents, preferably toluene and xylene and chlorinated solvents, preferably from chlorinated solvents.

10. Method according to any one of the claims 1-7, **characterized in that** the catalyst is lithium-trifluoromethanesulfonate and the solvent is chosen from organic solvents, preferably toluene and xylene, and chlorinated solvents, preferably dichloromethane, dichloroethane, chloroform and/or chlorobenzene.

11. Method according to any one of the claims 1-10, **characterized in that** the protecting groups of blocked monosaccharides or oligosaccharides are selected from (C₁-C₈)alkylcarbonyl, or optionally substituted phenylcarbonyl, or optionally substituted benzylcarbonyl, preferably from (C₁-C₄)alkylcarbonyl, or optionally substituted phenylcarbonyl, preferably phenylcarbonyl, tolylcarbonyl, xylylcarbonyl or benzylcarbonyl; and preferably is acetyl or p-chloro-phenylcarbonyl.

12. Method according to any one of the claims 1-11, **characterized in that** the substituents 1-0-acyl, 1-0-alkyl and 1-halogen attached to the blocked monosaccharide or oligosaccharide are preferably substituents of the formulae -O-(C₁-C₈)acyl, -O-(C₁-C₈)alkyl or halogen, preferably chlorine.

13. Method according to claim 12, **characterized in that** the substituents 1-0-acyl, 1-0-alkyl and 1-halogen are -O-(C₁-C₄)acyl, -O-(C₁-C₄)alkyl or chlorine, preferably -O-C(O)CH₃ or chlorine, preferably chlorine.

14. Method according to any one of the claims 1-13, **characterized in that** the blocked monosaccharides or oligosaccharides are derived from ribose, 2-deoxyribose, arabinose, and glucose and wherein preferably all free hydroxyl groups are blocked with known protecting groups, preferably with the blocking groups selected from (C₁-C₈)alkylcarbonyl, or optionally substituted phenylcarbonyl, or optionally substituted benzylcarbonyl.

15. Method according to any one of the claims 1-14, **characterized in that** the protected nucleoside base is protected by a removable protecting group known per se, and is preferably protected by a trimethylsilyl (TMS)-group.

16. Method according to any one of the claims 1-15, **characterized in that** the nucleoside base is from the group uracil, cytosine, preferably 5-azacytosine, 6-azauracil, 2-thio-6-azauracil, thymine, and guanine, or a halogen derivative, preferably fluoro derivatives thereof.

17. Method according to any one of the claims 1-16, **characterized in that** in the nucleoside obtained, the sugar residue is linked to the nitrogen atom to form a beta-glycoside.

## Patentansprüche

1. Verfahren zur Herstellung einer freien Nukleosidverbindung, mit der Maßgabe, dass die Verbindung 2'-Desoxy-5-azacytidin (Decitabin) ausgeschlossen ist, durch Umsetzung eines Glykosiddonors, vorzugsweise eines 1-Halogenderivats, oder 1-0-Acyl, 1-0-Alkyl, oder eines Imidats, vorzugsweise eines Trichlormethylimidats, oder eines Thioalkylderivats, vorzugsweise eines Thiomethylderivats eines blockierten Monosaccharids oder Oligosaccharids, vorzugsweise Ribose- und 2-Desoxyribosederivate, wobei das Monosaccharid bzw. Oligosaccharid durch entfernbare Schutzgruppen blockiert ist, mit einer geschützten Nukleosidbase in einem geeigneten wasserfreien Lösungsmittel und in Gegenwart eines Katalysators, wodurch man eine blockierte Nukleosidverbindung erhält, und Entfernen der Schutzgruppen von der blockierten Nukleosidverbindung unter Erhalt der freien Nukleosidverbindung, **dadurch gekennzeichnet, dass** es sich bei dem Katalysator um ein Salz einer aliphatischen Sulfonsäure oder ein Salz einer fluorierten aliphatischen Sulfonsäure handelt.

2. Verfahren zur Herstellung einer blockierten Nukleosidverbindung, mit der Maßgabe, dass die blockierte Verbindung von 2'-Desoxy-5-azacytidin (Decitabin) ausgeschlossen ist, durch Umsetzung eines Glykosiddonors, vorzugsweise eines 1-Halogenderivats, oder 1-0-Acyl, 1-0-Alkyl, oder eines Imidats, vorzugsweise eines Trichlormethylimidats, oder eines Thioalkylderivats eines blockierten Monosaccharids oder Oligosaccharids, vorzugsweise Ribose- und 2-Desoxyribosederivate, wobei das Monosaccharid bzw. Oligosaccharid durch entfernbare Schutzgruppen blockiert ist, mit einer geschützten Nukleosidbase in einem geeigneten wasserfreien Lösungsmittel und in Gegenwart eines Katalysators, unter Erhalt einer blockierten Nukleosidverbindung, **dadurch gekennzeichnet, dass** es sich bei dem Katalysator um ein Salz einer aliphatischen Sulfonsäure oder ein Salz einer fluorierten aliphatischen Sulfonsäure handelt.

3. Verfahren nach Anspruch 1 oder 2, **dadurch gekennzeichnet, dass** es sich bei dem in der Umsetzung verwendeten Katalysator um ein Salz von Methylsulfonsäure oder von Ethylsulfonsäure oder ein Salz von Trifluormethansulfonsäure, Pentafluorethylsulfonsäure oder Heptafluorpropylsulfonsäure handelt.

4. Verfahren nach Anspruch 3, **dadurch gekennzeichnet, dass** es sich bei dem Katalysator um ein Salz von Methylsulfonsäure und/oder ein Salz von Trifluormethansulfonsäure handelt.

5. Verfahren nach einem der Ansprüche 1-4, **dadurch gekennzeichnet, dass** es sich bei dem Katalysator um ein Alkalisalz oder ein Erdalkalisalz, vorzugsweise ein Salz von Lithium, Natrium, Kalium oder Magnesium, bevorzugt ein Lithiumsalz, handelt.

6. Verfahren nach einem der Ansprüche 1-5, **dadurch gekennzeichnet, dass** es sich bei dem Katalysator um Lithiummethylsulfonsäure und/oder Lithiumtrifluormethansulfonat handelt.

7. Verfahren nach einem der Ansprüche 1-4, **dadurch gekennzeichnet, dass** der Katalysator aus den Salzen von Scandium, vorzugsweise Sc(OTf)₃, oder von Kupfer, vorzugsweise Cu(OTf)₂, oder von Magnesium, vorzugsweise Mg(OTf)₂, umfassenden Salzen ausgewählt ist.

8. Verfahren nach einem der Ansprüche 1-7, **dadurch gekennzeichnet, dass** das Lösungsmittel zum Durchführen der Reaktion aus der organische Lösungsmittel, vorzugsweise Benzol, Toluol, Xylol, oder chlorierte Lösungsmittel, vorzugsweise Dichlormethan, Dichlorethan, Chloroform, Chlorbenzol, oder Toluol, Xylol, oder Acetonitril, Propylencarbonat und verwandte Lösungsmittel, umfassenden Gruppe ausgewählt ist.

9. Verfahren nach Anspruch 8, **dadurch gekennzeichnet, dass** das Lösungsmittel zum Durchführen der Reaktion aus organischen Lösungsmitteln, vorzugsweise Toluol und Xylol, und chlorierten Lösungsmitteln, vorzugsweise aus chlorierten Lösungsmitteln, ausgewählt ist.

10. Verfahren nach einem der Ansprüche 1-7, **dadurch gekennzeichnet, dass** es sich bei dem Katalysator um Lithiumtrifluormethansulfonat handelt und das Lösungsmittel aus organischen Lösungsmitteln, vorzugsweise Toluol und Xylol, und chlorierten Lösungsmitteln, vorzugsweise Dichlormethan, Dichlorethan, Chloroform und/oder Chlorbenzol, ausgewählt ist.

11. Verfahren nach einem der Ansprüche 1-10, **dadurch gekennzeichnet, dass** die Schutzgruppen von blockierten Monosacchariden oder Oligosacchariden aus (C₁-C₈)-Alkylcarbonyl oder gegebenenfalls substituiertem Phenylcarbonyl oder gegebenenfalls substituiertem Benzylcarbonyl, vorzugsweise aus (C₁-C₄)-Alkylcarbonyl oder gegebenenfalls substituiertem Phenylcarbonyl, bevorzugt Phenylcarbonyl, Tolylcarbonyl, Xylylcarbonyl oder Benzylcarbonyl, ausgewählt sind und vorzugsweise Acetyl oder p-Chlorphenylcarbonyl sind.

12. Verfahren nach einem der Ansprüche 1-11, **dadurch gekennzeichnet, dass** die Substituenten 1-0-Acyl, 1-0-Alkyl und 1-Halogen, die an das blockierte Monosaccharid oder Oligosaccharid gebunden sind, vorzugsweise Substituenten der Formel -O-(C₁-C₈)-Acyl, -O-(C₁-C₈)-Alkyl bzw. Halogen, bevorzugt Chlor, sind.

13. Verfahren nach Anspruch 12, **dadurch gekennzeichnet, dass** die Substituenten 1-0-Acyl, 1-0-Alkyl und 1-Halogen -O-(C₁-C₄)-Acyl, -O-(C₁-C₄)-Alkyl bzw. Chlor, vorzugsweise -O-C(O)CH₃ oder Chlor, bevorzugt Chlor, sind.

14. Verfahren nach einem der Ansprüche 1-13, **dadurch gekennzeichnet, dass** die blockierten Monosaccharide oder Oligosaccharide von Ribose, 2-Desoxyribose, Arabinose und Glukose abgeleitet sind, wobei vorzugsweise alle freien Hydroxylgruppen durch bekannte Schutzgruppen blockiert sind, vorzugsweise mit den Blockiergruppen ausgewählt aus (C₁-C₈)-Alkylcarbonyl oder gegebenenfalls substituiertem Phenylcarbonyl oder gegebenenfalls substituiertem Benzylcarbonyl.

15. Verfahren nach einem der Ansprüche 1-14, **dadurch gekennzeichnet, dass** die geschützte Nukleosidbase durch eine an sich bekannte entfernbare Schutzgruppe geschützt ist und vorzugsweise durch eine Trimethylsilyl(TMS)-Gruppe geschützt ist.

16. Verfahren nach einem der Ansprüche 1-15, **dadurch gekennzeichnet, dass** die Nukleosidbase aus der Gruppe Uracyl, Cytosin, vorzugsweise 5-Azacytosin, 6-Azauracil, 2-Thio-6-azauracil, Thymin und Guanin oder einem Halogenderivat, vorzugsweise Fluorderivaten davon stammt.

17. Verfahren nach einem der Ansprüche 1-16, **dadurch gekennzeichnet, dass** bei dem erhaltenen Nukleosid der Zuckerrest unter Bildung eines beta-Glykosids an das Stickstoffatom gebunden ist.

## Revendications

1. Procédé de production d'un composé nucléosidique libre, à condition que le composé 2'-désoxy-5-azacytidine (Decitabine) soit exclu, par réaction d'un donneur de glycoside, de préférence un dérivé 1-halogéné, ou 1-0-acyle, 1-0-alkyle, ou un imidate, de préférence un imidate de trichlorométhyle, ou un dérivé de thioalkyle, de préférence un dérivé thiométhylique d'un monosaccharide ou oligosaccharide bloqué, de préférence des dérivés de ribose et 2-désoxyribose, ledit monosaccharide ou oligosaccharide étant bloqué par des groupes protecteurs labiles, avec une base nucléosidique protégée, dans un solvant anhydre adapté et en présence d'un catalyseur, de telle manière qu'un composé nucléosidique bloqué soit obtenu, et élimination des groupes protecteurs dudit composé nucléosidique bloqué afin d'obtenir le composé nucléosidique libre, **caractérisé en ce que** ledit catalyseur est un sel d'un acide sulfonique aliphatique ou un sel d'un acide sulfonique aliphatique fluoré.

2. Procédé de production d'un composé nucléosidique bloqué, à condition que le composé bloqué de 2'-désoxy-5-azacytidine (Decitabine) soit exclu, par réaction d'un donneur de glycoside, de préférence un dérivé 1-halogéné, ou 1-0-acyle, 1-0-alkyle, ou un imidate, de préférence un imidate de trichlorométhyle, ou un dérivé de thioalkyle d'un monosaccharide ou oligosaccharide bloqué, de préférence des dérivés de ribose et 2-désoxyribose, ledit monosaccharide ou oligosaccharide étant bloqué par des groupes protecteurs labiles, avec une base nucléosidique protégée, dans un solvant anhydre adapté et en présence d'un catalyseur, de sorte qu'un composé nucléosidique bloqué soit obtenu, **caractérisé en ce que** ledit catalyseur est un sel d'un acide sulfonique aliphatique ou un sel d'un acide sulfonique aliphatique fluoré.

3. Procédé selon la revendication 1 ou 2, **caractérisé en ce que** le catalyseur utilisé dans ladite réaction est un sel d'acide méthylsulfonique ou d'acide éthylsulfonique, ou un sel d'acide trifluorométhanesulfonique, d'acide pentafluoroéthylsulfonique, ou d'acide heptafluoropropylsulfonique.

4. Procédé selon la revendication 3, **caractérisé en ce que** le catalyseur est un sel d'acide méthylsulfonique et/ou un sel d'acide trifluorométhanesulfonique.

5. Procédé selon l'une quelconque des revendications 1 à 4, **caractérisé en ce que** le catalyseur est un sel de métal alcalin ou un sel de métal alcalino-terreux, de préférence un sel de lithium, sodium, potassium, ou magnésium, de préférence un sel de lithium.

6. Procédé selon l'une quelconque des revendications 1 à 5, **caractérisé en ce que** le catalyseur est lithium-acide méthylsulfonique et/ou lithium-trifluorométhanesulfonate.

7. Procédé selon les revendications 1 à 4, **caractérisé en ce que** le catalyseur est choisi parmi les sels comprenant des sels de scandium, de préférence Sc(OTf)₃, ou de cuivre, de préférence Cu(OTf)₂ ou de magnésium, de préférence Mg(OTf)₂.

8. Procédé selon l'une quelconque des revendications 1 à 7, **caractérisé en ce que** le solvant pour conduire la réaction est choisi dans le groupe comprenant des solvants organiques, de préférence le benzène, le toluène, le xylène, ou des solvants chlorés, de préférence le dichlorométhane, le dichloroéthane, le chloroforme, le chlorobenzène, ou le toluène, le xylol, ou l'acétonitrile, le carbonate de propylène et des solvants associés.

9. Procédé selon la revendication 8, **caractérisé en ce que** le solvant pour conduire la réaction est choisi parmi des solvants organiques, de préférence le toluène et le xylène et des solvants chlorés, de préférence parmi des solvants chlorés.

10. Procédé selon l'une quelconque des revendications 1 à 7, **caractérisé en ce que** le catalyseur est lithium-trifluorométhanesulfonate et le solvant est choisi parmi des solvants organiques, de préférence le toluène et le xylène, et des solvants chlorés, de préférence le dichlorométhane, le dichloroéthane, le chloroforme et/ou le chlorobenzène.

11. Procédé selon l'une quelconque des revendications 1 à 10, **caractérisé en ce que** les groupes protecteurs de monosaccharides ou oligosaccharides bloqués sont choisis parmi (alkyle en C₁-C₈)carbonyle, ou phénylcarbonyle facultativement substitué, ou benzylcarbonyle facultativement substitué, de préférence parmi (alkyle en C₁-C₄)carbonyle, ou phénylcarbonyle facultativement substitué, de préférence phénylcarbonyle, tolylcarbonyle, xylylcarbonyle ou benzylcarbonyle ; et sont de préférence acétyle ou p-chloro-phénylcarbonyle.

12. Procédé selon l'une quelconque des revendications 1 à 11, **caractérisé en ce que** les substituants 1-0-acyle, 1-0-alkyle et 1-halogène liés au monosaccharide ou oligosaccharide bloqué sont de préférence des substituants des formules -0-(acyle en C₁-C₈), -O-(alkyle en C₁-C₈) ou halogène, de préférence chlore.

13. Procédé selon la revendication 12, **caractérisé en ce que** les substituants 1-0-acyle, 1-0-alkyle et 1-halogène sont -O-(acyle en C₁-C₄), -O-(alkyle en C₁-C₄) ou chlore, de préférence -O-C(O)CH₃ ou chlore, de préférence chlore.

14. Procédé selon l'une quelconque des revendications 1 à 13, **caractérisé en ce que** les monosaccharides ou oligosaccharides bloqués sont dérivés de ribose, 2-désoxyribose, arabinose, et glucose et où, de préférence, tous les groupes hydroxyle sont bloqués par des groupes protecteurs connus, de préférence avec les groupes bloquants choisis parmi (alkyle en C₁-C₈) carbonyle, ou phénylcarbonyle facultativement substitué, ou benzylcarbonyle facultativement substitué.

15. Procédé selon l'une quelconque des revendications 1 à 14, **caractérisé en ce que** la base nucléosidique protégée est protégée par un groupe protecteur amovible connu en tant que tel, et est de préférence protégée par un groupe triméthylsilyle (TMS).

16. Procédé selon l'une quelconque des revendications 1 à 15, **caractérisé en ce que** la base nucléosidique est du groupe de l'uracile et la cytosine, de préférence 5-azacytosine, 6-azauracile, 2-thio-6-azauracile, thymine, et guanine, ou un dérivé halogéné, de préférence des dérivés fluorés de celles-ci.

17. Procédé selon l'une quelconque des revendications 1 à 16, **caractérisé en ce que** dans le nucléoside obtenu, le résidu glucidique est lié à l'atome d'azote pour former un bêta-glycoside.
